# EUROPEAN PATENT SPECIFICATION

(11) **EP 3 558 283 B1**
(45) Date of publication and mention of the grant of the patent: **16.11.2022**
(21) Application number: 16924736.8
(22) Date of filing: 22.12.2016
(51) Int. Cl.: A61K 31/05, C07C 39/21, B01D 9/00, B01D 11/02, A61K 36/60, C07C 37/84

(54) **METHOD FOR EXTRACTION OF OXYRESVERATROL FROM ARTOCARPUS HIRSUTUS**
VERFAHREN ZUR EXTRAKTION VON OXYRESVERATROL AUS ARTOCARPUS HIRSUTUS
MÉTHODE D'EXTRACTION D'OXYRESVÉRATROL À PARTIR D'ARTOCARPUS HIRSUTUS

(43) Date of publication of application: 30.10.2019
(73) Proprietor: Majeed, Muhammed, East Windsor, NJ 08520 (US); Nagabhushanam, Kalyanam, East Windsor, NJ 08520 (US); Nayak, Mahadeva, Bangalore 560058 (IN); Ananthanarayanan, Nagarajan, Bangalore 560058 (IN)
(72) Inventor: Majeed, Muhammed, East Windsor, NJ 08520 (US); Nagabhushanam, Kalyanam, East Windsor, NJ 08520 (US); Nayak, Mahadeva, Bangalore 560058 (IN); Ananthanarayanan, Nagarajan, Bangalore 560058 (IN)
(74) Representative: Dr. Gassner & Partner mbB
(86) International application number: PCT/US2016/068161
(87) International publication number: WO 2018/118059

(56) References cited:
- WO-A1-2016/056029
- CN-A- 105 418 381
- CN-B- 102 942 455
- KR-A- 20140 094 394
- US-A1- 2006 216 253
- US-A1- 2011 021 640
- US-A1- 2015 079 207
- US-B1- 7 253 324
- Pimporn Leelapornpisid ET AL: "Antiglycation and antioxidant activities of oxyresveratrol extracted from the heartwood of Artocarpus lakoocha Roxb", Maejo International Journal of Science and Technology, 1 November 2010 (2010-11-01), pages 454-461, XP055240117, Retrieved from the Internet: URL:http://www.mijst.mju.ac.th/vol4/454-46 1.pdf [retrieved on 2016-01-11]
- GAUTAM ET AL.: 'Artocarpus Lakoocha Roxb: An Overview' EUROPEAN JOURNAL OF COMPLEMENTARY AND ALTERNATIVE MEDICINE vol. 1, no. 1, 08 November 2013, pages 10 - 14, XP055240285
- LORENZ ET AL.: 'Oxyresveratrol and resveratrol are potent antioxidants and free radical -scavengers: effect on nitrosative and oxidative stress derived from microglial cells' NITRIC OXIDE vol. 9, no. 2, September 2003, pages 64 - 76, XP055512886
- SUN H. Y. ET AL.: 'Efficient Synthesis of Natural Polyphenolic Stilbenes: Resveratrol, Piceatannol and Oxyresveratrol' CHEMICAL AND PHARMACEUTICAL BULLETIN vol. 58, no. 11, 01 November 2010, pages 1492 - 1496, XP055203415

## Description

### FIELD OF THE INVENTION

The present invention relates to a method for extraction of Oxyresveratrol from *Artocarpus hirsutus (A. hirsutus).*

### BACKGROUND OF THE INVENTION

### DESCRIPTION OF PRIOR ART

Oxyresveratrol is a hydroxyl-substituted stilbene found in the roots, leaves, stem and fruit of many widely distributed plants including Moraceae, Liliaceae, Gnetaceae, etc. The biological activity of this compound is well documented in the following scientific articles;
1. Li Xu, Chao Liu, Wei Xiang, Hu Chen, Xiaoli Qin and Xianzhi Huang, Advances in the Study of Oxyresveratrol, International Journal of Pharmacology, 2014, 10, 44-54
2. Likhitwitayawuid, K., Sornsute, A., Sritularak, B., Ploypradith, P., Chemical transformations of oxyresveratrol (trans-2,4,3',5'-tetrahydroxystilbene) into a potent tyrosinase inhibitor and a strong cytotoxic agent. (Bioorganic & Medicinal Chemistry Letters, 2006, 16, 5650-5653)

It is also reported for its modest inhibition of HIV (Likhitwitayawuid K, Sritularak B, Benchanak K, Lipipun V, Mathew J, Schinazi RF, Phenolics with antiviral activity from Millettia erythrocalyx and Artocarpus lakoocha, Nat Prod Res. 2005 Feb; 19(2):177-82).

Oxyresveratrol has been isolated from several *Artocarpus* species (*A. lakoocha* Roxb.; *A. champlasha* Roxb.; *A. champlasha* Roxb.; *A. heterophyllus*; *A. gomezianus* Wall.). (Nasapon Povichit, Ampai Phrutivorapongkul, Maitree Suttajit and Pimporn Leelapornpisid, Antiglycation and antioxidant activities of Oxyresveratrol extracted from the heartwood of Artocarpus lakoocha Roxb., Maejo Int. J. Sci. Technol. 2010, 4(03), 454-461) using chromatographic techniques like HPLC, Column chromatography etc. While these techniques are expensive and time consuming, a simple inexpensive and reliable method of isolation is technically desired for the large scale industrial production of Oxyresveratrol. The present invention solves such technical problems by disclosing a simple solvent-solvent purification method of Oxyresveratrol from *A. hirsutus* with high recovery.

Further relevant prior art is found in KR 2014 0094394 A, CN 102 942 455 B and US 2006/216253 A1.

It is the principle objective of the invention to disclose a simple, commercially viable method for the extraction of Oxyresveratrol from *Artocarpus hirsutus.*

The present invention fulfils the aforesaid objectives and provides further related advantages.

### SUMMARY OF THE INVENTION

Disclosed is method for extraction of Oxyresveratrol from *A. hirsutuis.* More specifically, the invention discloses a novel method for extraction of Oxyresveratrol from *A. hirsutus* using a simple solvent-solvent purification method.

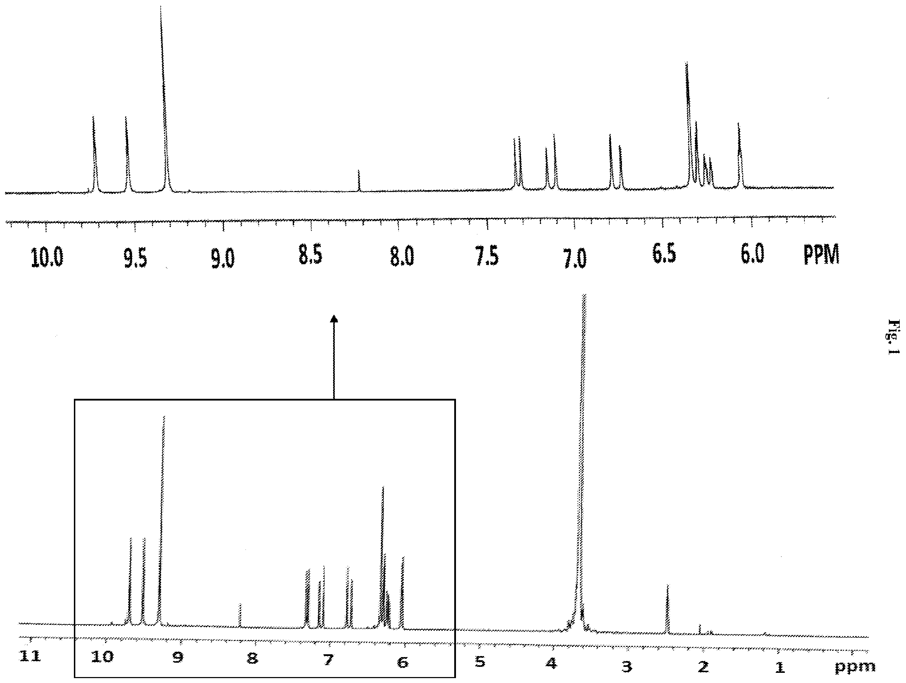

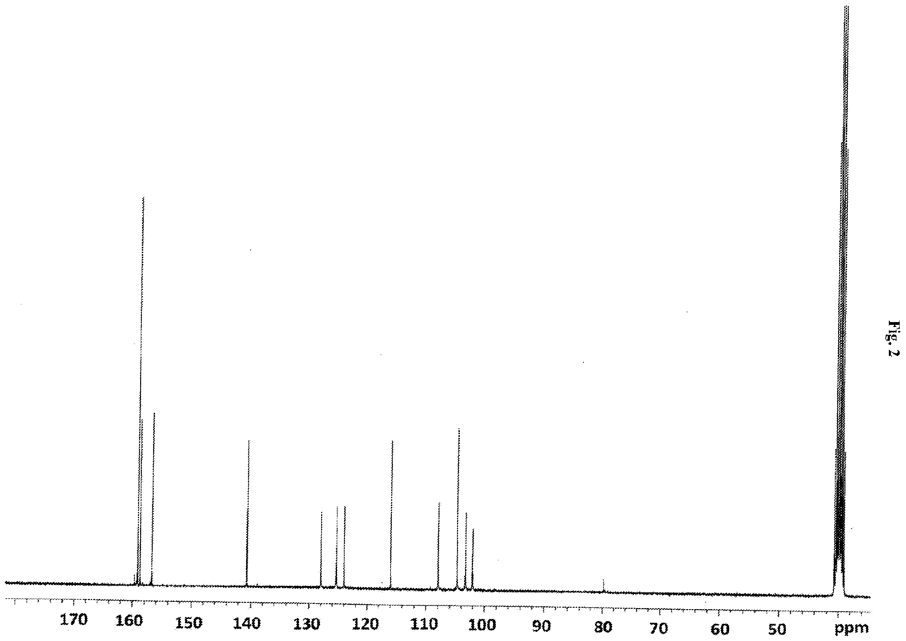

### BRIEF DESCRIPTION OF THE DRAWINGS (Figure 1 and 2)

**Fig. 1** shows ¹H-NMR (DMSO-*d₆*, 300 MHz): δ 9.72 (1H, s, 5-OH), 9.54 (1H, s, OH), 9.32 (2H, s, OH), 7.33 (1H, d, J=8.7 Hz, H-6), 7.14 (1H, d, J= 16.5 Hz, H-α), 6.76 (1H, d, J= 16.5 Hz, H-β), 6.34 (2H, d, J=2.1 Hz. H-2' and H-6'), 6.30 (1H, d, J=2.4 Hz. H-3), 6.24 (1H, dd, J=8.4 and 2.1 Hz, H-5), 6.06 (1H, t, J=2.1 Hz,, H-4').
**Fig. 2** shows ¹³C NMR of Oxyresveratrol (DMSO-*d₆*, 75 MHz): δ 158.75 (C-3' and 5'), 158.37 (C-4), 156.30 (C-2), 140.36 (C-1'), 127.60 (C-6), 124.97 (C-β), 123.57 (C-α), 115.61 (C-1), 107.60 (C-5), 104.34 (C-2' and 6'), 102.87 (C-4'), 101.70 (C-3).

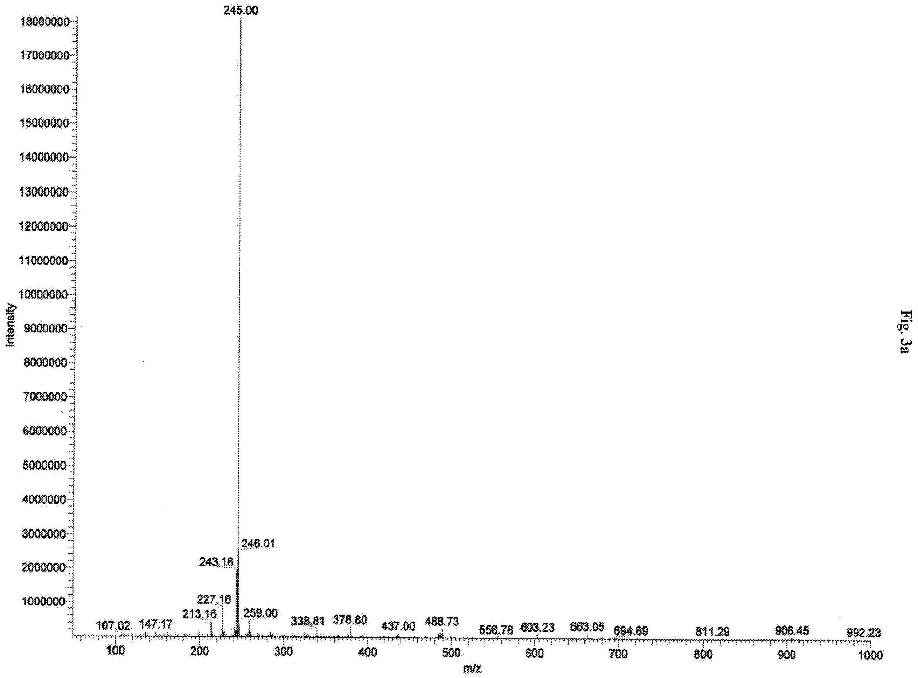

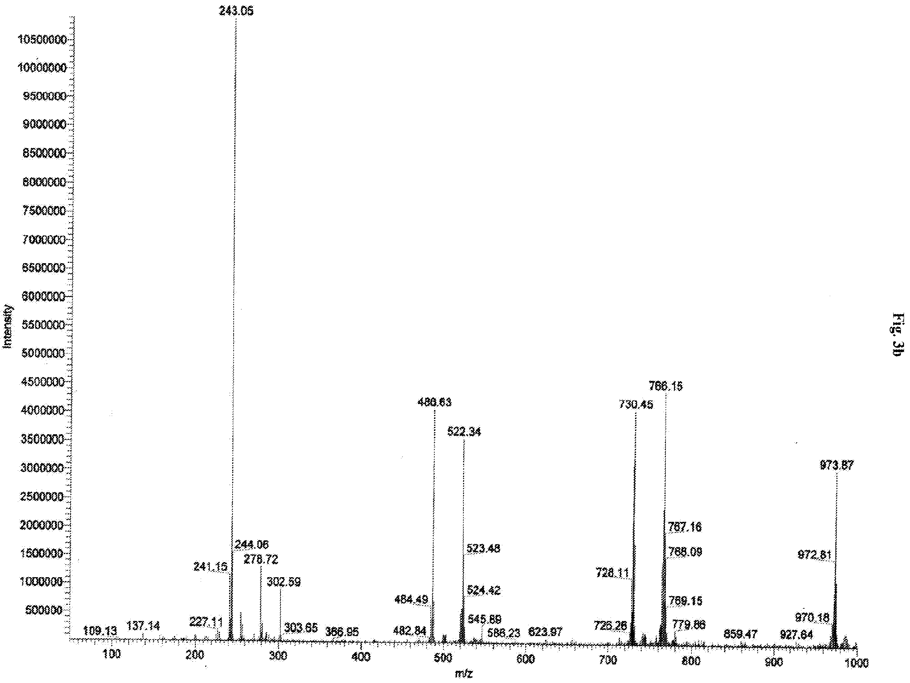

**Fig. 3a** **and** **3b** shows Liquid Chromatography Mass Spectrometry (LCMS) of compound Oxyresveratrol obtained from *A. hirsutus*

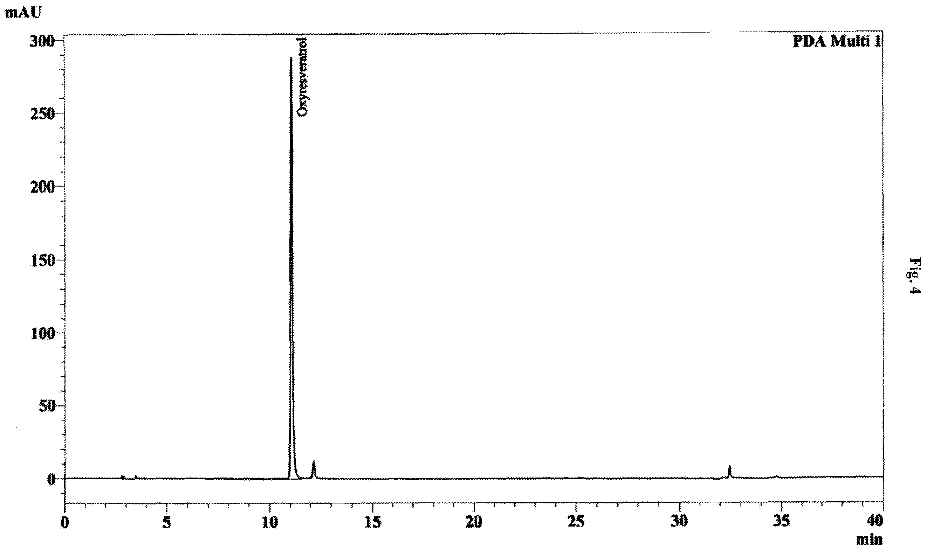

**Fig. 4** shows High Performance Liquid Chromatography (HPLC) of compound Oxyresveratrol obtained from *A. hirsutus*

### DETAILED DESCRIPTION OF THE MOST PRFFRED EMBODIMENT

In the most preferred embodiment, the present invention discloses a novel method for extraction of Oxyresveratrol from *Artocarpus hirsutus,* said method comprising steps of:
a) Cutting, drying the wood of *A. hirsutus* and pulverising to coarse powder;
b) Extracting powdered material from step a with hot ethanol (9 volumes) completely
c) Filtering to separate the ethanol extract from step b;
d) Concentrating the extract from step c under vacuum at 50-55 °C to thick paste;
e) Drying the extract from step d completely in vacuum tray dryer at 65-70 °C to get powder;
f) Dissolving the powder from step e in ethanol (2 volumes);
g) Addition of the ethanolic extract from step f slowly into 10 volumes of water under continuous stirring;
h) Separating the insolubles obtained from step g to get clear layer by filtration;
i) Washing the clear layer from step h with chloroform (1 volume) and discard the chloroform layer
j) Extracting the clear layer from step i with ethyl acetate (1 volume)
k) Concentrating the ethyl acetate layer from step j under vacuum at 50-55 °C to thick paste;
l) Drying the concentrated extract from step k to get powder in vacuum tray dryer at 65-70 °C;
m) Dissolving the powder from step 1 in water (4 volumes) and heating upto 80-90 °C for 8 h under stirring;
n) Cool the water layer from step m to 10-15 °C for 8 h and allowed for crystallization;
o) Filtering the crystals obtained from step n;
p) Drying the crystals from step o in a vacuum at 70-75 °C;
q) Characterizing the crystals from step p as Oxyresveratrol from its ¹H and ¹³C NMR spectra as well as from its LCMS spectrum as represented in STR#1

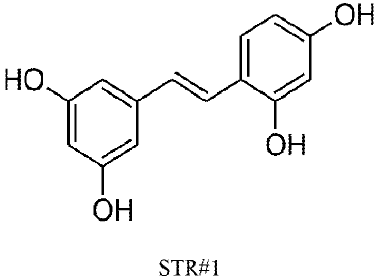

Other features and advantages of the present invention will become apparent from the following more detailed description, taken in conjunction with the accompanying images, which illustrate, by way of example, the principle of the invention.

### Example

### Preparation of the extract

*Artocarpus hirsutus* wood was collected and cut into small pieces, dried in shade. The dried material was pulverised to a coarse powder and stored in an air tight container. The powdered material was extracted completely with soaked volume of hot ethanol (3 volumes x 3 times). The ethanolic extracts were separated by filtration and collected in a clean container. The combined extracts were concentrated to thick paste under vacuum at 50-55 °C and dried in vacuum tray drier to get powder. The powdered extract was collected and stored at room temperature in air tight container.

### Isolation and characterization of active compound

The powdered ethanolic extract from *A. hirsutus* wood was redissolved in a small amount (2 volumes) of ethanol and poured into 10 volumes of water. The material was filtered to separate the water soluble and water insoluble fraction. The water soluble fraction was then extracted with ethyl acetate (1 volume) and the ethyl acetate layer was dried completely to get the powder. The powder obtained from ethyl acetate fraction was then poured into water (4 volumes) and stirred at 80-90 °C for 8 h. After cooling at room temperature, the solution was filtered and the crystallised solid material was dried under vacuum at 65 -70 °C. This filtered crystallised material was characterized as Oxyresveratrol from its ¹H and ¹³C NMR spectra as well as from its LCMS spectrum and was comparable with the reported values. The isolated Oxyresveratrol shows the following values: C₁₄H₁₂O₄. m. p.: 191-194 °C. Color: pale brown fine powder, APCI-MS *m*/*z* 245.00 (M+H⁺) and 245.05 (M-H⁻) (C₁₄H₁₂O₄ requires 244.2426). Purity of the compound was further verified by HPLC.

While the invention has been described with reference to a preferred embodiment, it is to be clearly understood by those skilled in the art that the invention is not limited thereto. Rather, the scope of the invention is to be interpreted only in conjunction with the appended claims.

## Claims

1. A novel method for extraction of Oxyresveratrol from *Artocarpus hirsutus,* said method comprising steps of:
a) Cutting, drying the wood of *A. hirsutus* and pulverising to coarse powder;
b) Extracting powdered material from step a with 9 volumes hot ethanol completely
c) Filtering to separate the ethanol extract from step b;
d) Concentrating the extract from step c under vacuum at 50-55 °C to thick paste;
e) Drying the extract from step d completely in vacuum tray dryer at 65-70 °C to get powder;
f) Dissolving the powder from step e in 2 volumes ethanol;
g) Addition of the ethanolic extract from step f slowly into 10 volumes of water under continuous stirring;
h) Separating the insolubles obtained from step g to get clear layer by filtration;
i) Washing the clear layer from step h with 1 volume chloroform and discard the chloroform layer
j) Extracting the clear layer from step i with 1 volume ethyl acetate
k) Concentrating the ethyl acetate layer from step j under vacuum at 50-55 °C to thick paste;
l) Drying the concentrated extract from step k to get powder in vacuum tray dryer at 65-70 °C;
m) Dissolving the powder from step 1 in 4 volumes water and heating upto 80-90 °C for 8 h under stirring;
n) Cool the water layer from step m to 10-15 °C for 8 h and allowed for crystallization;
o) Filtering the crystals obtained from step n;
p) Drying the crystals from step o in a vacuum at 70-75 °C;
q) Characterizing the crystals from step p as Oxyresveratrol from its ¹H and ¹³C NMR spectra as well as from its LCMS spectrum as represented in STR#1

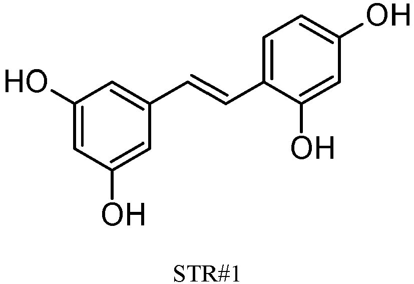

## Patentansprüche

1. Ein neues Verfahren zur Extraktion von Oxyresveratrol aus *Artocarpus hirsutus,* wobei das Verfahren die folgenden Schritte umfasst:
a) Schneiden, Trocknen des Holzes von *A. hirsutus* und Zermahlen zu grobem Pulver;
b) vollständiges Extrahieren des pulverförmigen Materials aus Schritt a mit 9 Volumen heißem Ethanol
c) Filtrieren, um den Ethanolextrakt aus Schritt b abzutrennen;
d) Konzentrieren des Extrakts aus Schritt c unter Vakuum bei 50-55 °C zu einer dicken Paste;
e) vollständiges Trocknen des Extrakts aus Schritt d in einem Vakuum-Hordentrockner bei 65-70 °C zur Gewinnung von Pulver;
f) Auflösen des Pulvers aus Schritt e in 2 Volumen Ethanol;
g) Langsames Zugeben des ethanolischen Extrakts aus Schritt f in 10 Volumen Wasser unter kontinuierlichem Rühren;
h) Abtrennen des in Schritt g erhaltenen Unlöslichen durch Filtration, um eine klare Schicht zu erhalten;
i) Waschen der klaren Schicht aus Schritt h mit einem Volumen Chloroform und Verwerfen der Chloroformschicht
j) Extrahieren der klaren Schicht aus Schritt i mit einem Volumen Ethylacetat
k) Konzentrieren der Ethylacetatschicht aus Schritt j unter Vakuum bei 50-55 °C zu einer dicken Paste;
l) Trocknen des konzentrierten Extrakts aus Schritt k zur Gewinnung von Pulver in einem Vakuum-Hordentrockner bei 65-70 °C;
m) Auflösen des Pulvers aus Schritt l in 4 Volumen Wasser und Erhitzen auf 80-90 °C für 8 Stunden unter Rühren;
n) Kühlen der Wasserschicht aus Schritt m auf 10-15 °C für 8 Stunden und Zulassen einer Kristallisation;
o) Filtern der in Schritt n erhaltenen Kristalle;
p) Trocknen der Kristalle aus Schritt o im Vakuum bei 70-75 °C;
q) Charakterisieren der Kristalle aus Schritt p als Oxyresveratrol anhand seiner ¹H und ¹³C NMR-Spektren sowie anhand seines LCMS-Spektrums, wie in STR#1 dargestellt

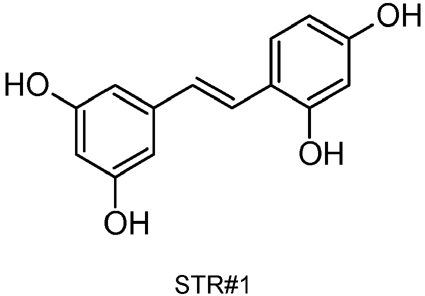

## Revendications

1. Nouveau procédé d'extraction d'oxyresvératrol de *Artocarpus hirsutus,* ledit procédé comprenant les étapes consistant à :
a) couper, sécher le bois de *A. hirsutus* et le pulvériser en poudre grossière ;
b) extraire du matériau pulvérisé de l'étape a avec 9 volumes d'éthanol chaud complètement
c) filtrer pour séparer l'extrait d'éthanol de l'étape b ;
d) concentrer l'extrait de l'étape c sous vide à 50 à 55 °C en une pâte épaisse ;
e) sécher l'extrait de l'étape d complètement dans une étuve à plateaux sous vide à 65 à 70 °C pour obtenir de la poudre ;
f) dissoudre la poudre de l'étape e dans 2 volumes d'éthanol ;
g) addition de l'extrait éthanolique de l'étape f lentement dans 10 volumes d'eau sous agitation continue ;
h) séparer les insolubles obtenus à l'étape g pour obtenir une couche transparente par filtration ;
i) laver la couche transparente de l'étape h avec 1 volume de chloroforme et jeter la couche de chloroforme
j) extraire la couche transparente de l'étape i avec 1 volume d'acétate d'éthyle
k) concentrer la couche d'acétate d'éthyle de l'étape j sous vide à 50 à 55 °C en une pâte épaisse ;
l) sécher l'extrait concentré de l'étape k pour obtenir de la poudre dans une étuve à plateaux sous vide à 65 à 70 °C ;
m) dissoudre la poudre de l'étape l dans 4 volumes d'eau et chauffer jusqu'à 80 à 90 °C pendant 8 h sous agitation ;
n) refroidir la couche d'eau de l'étape m à 10 à 15 °C pendant 8 h et laisser cristalliser ;
o) filtrer les cristaux obtenus de l'étape n ;
p) sécher les cristaux de l'étape o sous vide à 70 à 75 °C ;
q) caractériser les cristaux de l'étape p en tant qu'oxyresvératrol à partir de ses spectres RMN ¹H et ¹³C ainsi que de son spectre CL-SM tel que représenté dans STR#1
